# EUROPEAN PATENT APPLICATION

(11) **EP 1 580 264 A1**
(43) Date of publication of application: **28.09.2005**
(21) Application number: 03769965.9
(22) Date of filing: 29.10.2003
(51) Int. Cl.: C12N 15/09, A01K 67/027, C12N 5/10

(54) **CONSTRUCTION OF KNOCKDOWN ANIMAL BY TRANSFERRING DOUBLE-STRANDED RNA EXPRESSION VECTOR**

(30) Priority: 29.10.2002 JP 2002314764
(71) Applicant: ORIENTAL YEAST CO., LTD., Tokyo 174-8505 (JP); RIKEN, Saitama 351-0198 (JP)
(72) Inventor: ISHII, Shunsuke, c/o Tsukuba Institute, Riken, Tsukuba-shi, Ibaraki 305-0074 (JP); SHINAGAWA, Toshie, c/o Tsukuba Institute, Riken, Tsukuba-shi, Ibaraki 305-0074 (JP); UCHIDA, Koji, c/o Oriental Yeast Co., Ltd., Itabashi-ku, Tokyo 174-8505 (JP); HAYASHI, Naoki, c/o Oriental Yeast Co., Ltd., Itabashi-ku, Tokyo 174-8505 (JP)
(74) Representative: Hinkelmann, Klaus
(86) International application number: PCT/JP2003/013855
(87) International publication number: WO 2004/039973

(57) **Abstract**

This invention relates to a target gene-knockdown animal and an animal model for disease in which mRNA of an endogenous target gene is disrupted via formation of double-stranded RNA for the target gene and a method for producing the animals. Also, this invention relates to a ds-RNA expression vector used for such method and an animal cell having such vector introduced therein.

## Description

### Technical Field

The present invention relates to a target gene-knockdown animal, an animal model for disease in which mRNA of an endogenous target gene is disrupted via formation of double-stranded RNA (ds-RNA) for the target gene and a method for producing such animals. Also, the present invention relates to a ds-RNA expression vector used for the aforementioned method and an animal cell into which such vector has been introduced.

### Background Art

Mice carrying specific gene mutations play key roles as murine models for disease in fields of applied science as well as in fields of basic science, such as embryology. Murine models for disease are very useful for the development of diagnostic techniques and therapeutic agents for various types of diseases. The method for producing mutant mice with the use of ES cells, which is generally employed at present, was developed via basic research conducted by Capecchi *et al.* (Capecchi, MR., Science (U.S.A.), 1989, vol. 244, pp. 1288-1292 (review)). In this technique, mutant mice are produced in the following procedures: 1) the genomic clone of the target gene is first separated, and a targeting vector in which the exon region essential for the functions has been substituted with a drug (e.g., neomycin)-resistant gene is constructed; 2) the resulting targeting vectors are introduced into ES cells by electroporation or other methods, and recombinant cells in which the genes of ES cells have been substituted with targeting vectors via homologous recombination are separated; 3) the resulting recombinant cells are introduced into 8-cell-stage embryos to prepare chimeric mice; and 4) mice in which germ cells derived from the ES cells are formed are selected from the resulting chimeric mice, and homozygous mutant mice are obtained via mating. Since the methods of producing mutant mice that are generally employed at present involve a large number of steps, it would generally take for about 1 year and enormous labor to carry out such methods.

A phenomenon referred to as "RNA interference" has drawn attention in recent years. This was originally found in, for example, nematodes (*C. elegans*), *Drosophila,* or plants. When double-stranded RNA (ds-RNA) corresponding to a specific mRNA sequence is introduced into a cell, mRNA transcribed in that cell is immediately disrupted (see Hannon, GJ., Nature, 2002, vol. 418, pp. 244-251 (review); JP Patent Publication (Kohyo) Nos. 2002-516062 A (WO 99/32619); 8-506734 A (1996); 2002-507416 A (WO 99/49029); and 2003-516124 (WO 01/29058)). In recent years, such RNA interference has been found to occur in cultured animal cells or even in mice (see Elbashir, SM. *et al.,* Nature, 2001, vol. 411, pp. 494-498; McCaffrey, AP. *et al*., Nature, 2002, vol. 418, pp. 38-39; JP Patent Publication (Kohyo) Nos. 2002-502012A (WO 00/44895) and 2003-514533 A (WO 01/36646)). A series of analyses that have been conducted up to the present demonstrate that ds-RNA introduced into a cultured cell or the like is degraded into small RNA of about 22 nucleotides, and such small RNA hybridizes to mRNA to induce degradation of mRNA (Tuschl, T. *et al*., Nature Biotech., 2002, vol. 20, pp. 446-448; JP Patent Publication (Kohyo) No. 2003-529374 A (WO 01/75164); and WO 02/44321). It has been confirmed by many experiments that degradation of mRNA is induced by long ds-RNA in the case of *Drosophila* or nematodes. In the case of animal cells, it has been confirmed that degradation of mRNA is induced by small ds-RNA of 20 to 30 nucleotides. It is also suggested that long ds-RNA disrupts a large number of mRNA molecules in a non-sequence-specific manner (Elbashir, SM. *et al*., Nature, 2001, vol. 411, pp. 494-498; and WO 00/175164).

A large number of polymerase-III based siRNA expression systems used in order to effect RNAi in animal cells have been reported (for example, Miyagishi, M. *et al*., Nature Biotechnol., 2002, vol. 20, pp. 497-500; and Hasuwa, H. *et al*., FEBS Lett., 2002, vol. 532, pp. 227-230). In the case of siRNA-based RNAi, however, it is difficult to select a target site, the expression at which is to be suppressed. Thus, there is no effective method of predication at this moment. Since polymerase-III promoters are active in all tissues, it is impossible to cause tissue-specific RNAi.

In the case of polymerase II-based RNAi in animal cells, it is pointed out that kinase is activated by ds-RNA upon viral infection or the like, and an interferon signal transduction pathway is activated (Kaufman, RJ. *et al*., Proc. Natl. Acad. Sci. U.S.A., 1999, vol. 96, pp. 11693-11695). When particularly long ds-RNA is expressed in animal cells during the production of transgenic animals as described above, it is also reported that protein synthesis is inhibited in a non-sequence-specific manner and a pathway inducing an interferon is activated (Elbashir, S.M. *et al*., Nature, 2001, vol. 411, pp. 494-498).

Accordingly, it is necessary to overcome the problems as mentioned above and to efficiently cause RNAi in animal cells.

### Summary of the Invention

It is an object of the present invention to provide a method for simply and rapidly producing target gene-knockdown animals and a tool therefor.

The present inventors have conducted concentrated studies in order to attain the above object. As a result, we considered that production of a transgenic animal that is capable of expressing ds-RNA for a target gene in a specific tissue would enable production of a target gene-knockdown animal in a simple and rapid manner. Thus, the present inventors selected the Ski transcription factor as a target gene, prepared a vector that would express 540 bp ds-RNA corresponding to the mRNA sequence of the Ski gene, and produced a transgenic mouse into which the resulting expression vector has been introduced. As a result, we confirmed that the aforementioned transgenic mouse exhibited abnormalities such as abnormal morphogenesis or hemorrhage during the developmental stage, as with the case of conventional Ski mutant mice. Therefore, the present inventors found that a target gene-knockdown mouse could be produced within a short period of time via utilization of RNA interference using the expression vector constructed in the aforementioned manner. This has led to the completion of the present invention.

More specifically, the present invention relates to a double-stranded RNA (ds-RNA) expression vector that comprises the following sequences (a) to (c):
(a) the following nucleotide sequence (a-1) or (a-2):
   (a-1) a nucleotide sequence encoding all or a part of the target gene; or
   (a-2) a nucleotide sequence encoding DNA that hybridizes under stringent conditions to DNA consisting of a sequence complementary to the nucleotide sequence (a-1);
(b) a nucleotide sequence complementary to the nucleotide sequence (a) and an inverted repeat thereof; and
(c) a sequence encoding a loop region and connecting the nucleotide sequence (a) to the nucleotide sequence (b),
wherein the above sequences are transcribed into RNA and thereby forming ds-RNA having a stem-loop structure.

The ds-RNA expression vector preferably comprises a polymerase II promoter or a developmental-stage-specific promoter. An example of such promoter is a cytomegalovirus (CMV) early gene promoter.

The ds-RNA expression vector preferably comprises a sequence that autocatalytically cleaves RNA located upstream of the nucleotide sequences (a) to (c). An example of such sequence is, but is not limited to, a ribozyme site.

The ds-RNA expression vector preferably comprises a sequence that pauses RNA polymerase located downstream of the nucleotide sequences (a) to (c). An example of such sequence is, but is not limited to, a sequence of the MAZ domain.

Examples of the nucleotide sequence (c) of the ds-RNA expression vector include those shown in SEQ ID NOs: 2, 5, and 6.

An example of the target gene of the ds-RNA expression vector is a disease-associated gene, and a specific example thereof is the Ski gene. A part of the target gene may be a 540 bp 5'-region of the Ski gene.

The present invention also relates to a target gene-knockdown animal in which ds-RNA for the target gene is expressed, preferably the ds-RNA for the target gene is tissue-specifically expressed.

Examples of the animal include a transgenic animal into which the aforementioned ds-RNA expression vector has been introduced and expressing ds-RNA for the target gene and a progeny thereof.

When the target gene is a disease-associated gene, for example, the animal can be an animal model for disease. When the Ski gene is selected as the target gene, the animal can be an animal model for disease exhibiting a disease selected from the group consisting of neural tube closure defect, malformation of the iris, and hemorrhage in the head.

An example of the animal is, but is not limited to, a mouse.

The present invention also relates to a method for producing a target gene-knockdown animal, wherein a ds-RNA expression vector that is capable of expressing ds-RNA for the target gene is introduced to form ds-RNA for the target gene.

In this method, either of the aforementioned ds-RNA expression vectors is preferably used.

When a disease-associated gene is selected as the target gene, an animal model for disease can be produced as the animal. When the Ski gene is selected as the target gene, for example, animal models of disease exhibiting a disease selected from the group consisting of neural tube closure defect, malformation of the iris, and hemorrhage in the head can be constructed.

In this method, a preferable example of such an animal is, but is not limited to, a mouse.

The present invention further relates to an animal cell into which the aforementioned ds-RNA expression vector has been introduced.

### Brief Description of the Drawings

Fig. 1A shows the structure of the Ski double-stranded (ds)-RNA expression vector.
Fig. 1B schematically shows a region encoding Ski ds-RNA.
Fig. 2 shows the predicted secondary structures of RNA transcribed from a variety of constructed plasmids.
Fig. 3A and 3B are photographs showing the results of Western blot analysis demonstrating a decrease in the Ski protein level in a cultured cell into which the Ski ds-RNA expression vector has been introduced.
Fig. 4 is a photograph showing the results of Northern blot analysis demonstrating the RNA level that is present in the nucleus and in the cytoplasm of a cell into which various types of Ski ds-RNA expression vectors have been introduced.
Fig. 5 is a photograph demonstrating that expression of long ds-RNA in a nucleus does not affect the phosphorylation of eIF2α.
Fig. 6A is a photograph showing the results of RT-PCR analyzing Ski mRNA degradation by a Ski ds-RNA expression vector.
Fig. 6B is a photograph showing the results of RT-PCR analyzing Ski mRNA degradation by a Ski ds-RNA expression vector over time.
Fig. 7A to 7C each independently show the effects of long ds-RNA expressed from the ds-RNA expression vector on luciferase activity. Fig. 7A shows *Photinus pyralis* luciferase (Pp-luc) activity plotted in arbitrary luminescence units (au). Fig. 7B shows *Renilla reniformis* luciferase (Rr-luc) activity plotted in arbitrary luminescence units. Fig. 7C shows the results concerning ratios of normalized target to control luciferase.
Fig. 8A is a photograph showing the results of Southern blot analysis demonstrating the presence of a transgene in a Ski ds-RNA transgenic mouse embryo.
Fig. 8B is a photograph showing the results of Northern blot analysis demonstrating the presence of the siRNA in a Ski ds-RNA transgenic mouse embryo.
Fig. 9 is a photograph showing neural tube closure defects of a Ski ds-RNA transgenic mouse.
Fig. 10 is a photograph showing malformation of the iris in an eye of a Ski ds-RNA transgenic mouse.
Fig. 11 is a photograph showing hemorrhage in the head of a Ski ds-RNA transgenic mouse.
Figs. 12A to 12G are photographs showing abnormalities caused by Ski RNA deficiency in a Ski ds-RNA transgenic mouse.

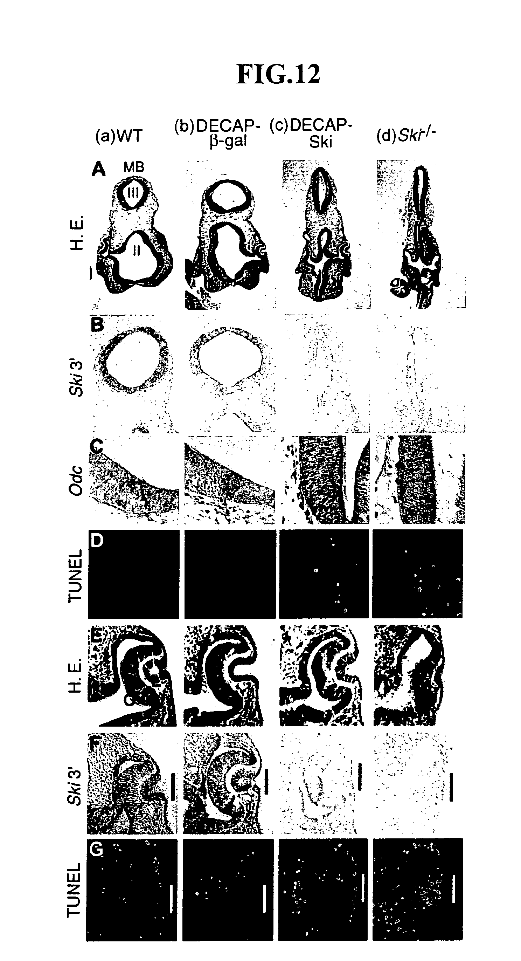

Fig. 12A shows hematoxylin-eosin staining of head sections.
Figs. 12B shows Ski mRNA expression in the midbrain and in the mesenchyme as detected by RNA *in situ* hybridization.
Fig. 12C shows Odc mRNA expression in the midbrain and in the mesenchyme as detected by RNA *in situ* hybridization.
Fig. 12D shows apoptotic cells assessed by the TUNEL assay on the neuroepithelium of the midbrain and the mesenchyme.
Fig. 12E shows hematoxylin-eosin staining of eyes.
Fig. 12F shows Ski mRNA expression in eyes as detected by *in situ* hybridization.
Fig. 12G shows the results of the TUNEL assay on eyes.

In Figs. 12A to 12G, "a" represents a wild-type mouse, "b" represents a pDECAP-β-gal transgenic mouse, "c" represents a pDECAP-Ski transgenic mouse, and "d" represents a Ski-deficient mouse (Ski^{-/-}). In Figs. 12A and 12E, "MB" represents the midbrain, "II" represents the second ventricle, "III" represents the third ventricle, "O" represents the optic cup, and "L" represents the lens vesicle.

### Preferred Embodiments of the Invention

The present invention is hereafter described in detail. This patent application claims priority from Japanese Patent Application No. 2002-314764 filed on October 29, 2002, and encompasses part or all of the contents as disclosed in the description and/or drawings thereof.

The present invention relates to a target gene-knockdown transgenic animal via RNA interference and a tool (a double-stranded RNA expression vector) and a method for simply and rapidly producing such transgenic animal.

The present inventors considered that selection of an adequate length for ds-RNA, an adequate promoter, and the like is important in order to allow ds-RNA for the target gene to express in a specific tissue when production of a transgenic animal is intended via RNA interference. Accordingly, an experiment was carried out using the transcription factor, Ski, for which the phenotype of the mutant mouse was analyzed in detail. As a result, the present inventors confirmed the expression of ds-RNA in transgenic animals. Since Ski mutation is known to cause various types of malformation during the fetal stage, it was possible to determine during the early developmental stage whether the target gene was knocked-down or not based on the expression and the formation of ds-RNA.

Hereafter, the target gene-knockdown animal, the animal model for disease according to the present invention, and a method for producing such animal models are described.

### 1. RNA interference

The "RNA interference" refers to the phenomenon described below. When double-stranded RNA (herein also referred to as "ds-RNA") is present in a cell, endogenous mRNA of a gene that is homologous with the nucleotide sequence of the RNA is degraded, and the gene expression in the cell is then suppressed in a specific manner. It is also referred to as RNA interference (RNAi). At first, RNA interference was found in nematodes and then observed in *Drosophila,* plants, mammalian cells, and the like. In the case of *Drosophila,* for example, a technique is known in which hairpin ds-RNA is constructed, the resultant is recognized as ds-RNA in the cell, and endogenous mRNA is then degraded and disrupted. A desired gene can be knocked down by setting such desired gene as the gene encoded by such hairpin ds-RNA. However, many of the mechanisms of RNA interference in a mammal are not yet revealed, RNAi is caused by trial and error at present, and this technique is not yet established as a generalized technique. When expression of ds-RNA is intended in a mammal, RNA interference is not always caused due to position effects or other conditions. Further, when particularly long ds-RNA is allowed to express in an animal cell, inhibition of protein synthesis in a non-sequence-specific manner or activation of an interferon-inducing pathway has been reported.

### 2. Construction of ds-RNA expression vector

The ds-RNA expression vector according to the present invention (hereinafter referred to as "the vector of the present invention") can be introduced into an animal cell and an animal so that ds-RNA for the target gene necessary for RNA interference is expressed and formed. In the present invention, "expression" refers to the phenomenon such that DNA encoding ds-RNA is transcribed into mRNA being capable of forming a ds-RNA, in the case of ds-RNA. In general, "expression" refers to the fact that DNA encoding a gene is transcribed into mRNA and mRNA is further translated into a protein. In the present invention, the term "expression" refers to both thereof. The term "ds-RNA for the target gene" refers to ds-RNA that induces RNA interference on the target gene. More specifically, it refers to ds-RNA having a sequence homologous to that of the target gene. Double-stranded RNA for this target gene is encoded on the vector of the present invention by the following sequences (a) to (c):
(a) the following nucleotide sequence (a-1) or (a-2):
   (a-1) a nucleotide sequence encoding all or a part of the target gene; or
   (a-2) a nucleotide sequence encoding DNA that hybridizes under stringent conditions to DNA consisting of a sequence complementary to the nucleotide sequence (a-1);
(b) a nucleotide sequence complementary to the nucleotide sequence (a) and an inverted repeat thereof; and
(c) a sequence encoding a loop region and connecting the nucleotide sequence (a) to the nucleotide sequence (b). Upon the above sequences are transcribed into RNA, double-stranded RNA (ds-RNA) having a stem-loop structure is formed. In the present invention, the term "stem-loop structure" refers to a structure consisting of a stem region that complementarily forms double strands and a loop region in a single stranded loop form that connects these two strands. Such structure is known in the field of molecular biology.

The nucleotide sequence (a) is a nucleotide sequence that encodes all or a part of the target gene to be knocked down (a-1). The target gene is not particularly limited as long as it is intended to be knocked down via RNA interference. Any gene that is deduced to be associated with disease or other functions, or that is to be studied can be employed as the target gene. Specific examples thereof include, but are not limited to, the Ski genes, other transcription factors, and cancer-associated genes.

The Ski gene was first identified as an oncogene, and functions thereof as a transcription corepressor was revealed in recent years (Nomura, T. *et al*., Genes and Development 13: 412-423, 1999). A transcription corepressor is a factor that represses transcription by binding to various types of repressors. One species thereof, Ski, plays key roles in various developmental stages. The Ski genes are isolated from humans, mice, frogs, and flies, the nucleotide sequences thereof are known and registered at GenBank (Accession Nos: X15218 (human), AF435852 (mouse), X68683 (frog), and NT_033778 (fly)).

In the present invention, the sequence (a) can be designed based on such publicly available nucleotide sequence information. Alternatively, a person skilled in the art can prepare DNA having the sequence (a) via a conventional technique if such DNA can be introduced into the ds-RNA expression vector without the need to know all the sequences. "A part of" the target gene refers to a sequence of a length that is sufficient for target gene-knockdown via RNA interference, such as a sequence of 500 to 1,000 bp, and preferably a sequence of 500 to 700 bp. When the Ski gene is employed, for example, a sequence of approximately 500 to 1,000 bp corresponding to the N-terminal region of the Ski protein is preferable. In the present invention, use of a 540 bp 5'-region (SEQ ID NO: 1) of the Ski gene as the nucleotide sequence (a) is particularly preferable. SEQ ID NO: 1 is derived from the mouse Ski gene (GenBank Accession No: AF435852) and corresponds to a sequence consisting of nucleotides 1 to 540 thereof. The vector of the present invention can express long ds-RNA of approximately 500 to 1,000 bp and can induce RNA interference. Thus, a ds-RNA expression vector can be simply and easily constructed without the need to select a region (of approximately 20 to 30 bp) for specifically degrading endogenous RNA, unlike the conventional case where siRNA (small interfering RNA) is employed.

The nucleotide sequence (a) also includes a nucleotide sequence (a-2) that hybridizes under stringent conditions to DNA consisting of a sequence complementary to the nucleotide sequence encoding all or a part of the target gene. Such nucleotide sequence is also capable of effectively causing RNA interference if it has a certain degree of homology to the target sequence. Under stringent conditions, a specific hybrid is formed but a non-specific hybrid is not formed. Under such conditions, for example, DNA having high homology (60% or higher and preferably 80% or higher) with other DNA is capable of hybridizing to the other DNA. More specifically, a sodium concentration is between 150 mM and 900 mM, and preferably between 600 mM and 900 mM, and temperature is between 60°C and 68°C, and preferably 65°C, under stringent conditions.

The nucleotide sequence (b) is complementary to the nucleotide sequence (a) and is an inverted repeat thereof. The term "complementary" used herein does not refer to precise complementarity. It would be sufficient if double strands could be complementarily formed with the nucleotide sequence (a). The "inverted repeat" concerns the direction of the sequence that is inserted into a vector. When the nucleotide sequence (a) is inserted in the form of ATCGAGTC, for example, the nucleotide sequence (b) is inserted in the form of GACTCGAT.

The nucleotide sequence (c) encodes a loop region, and it connects the nucleotide sequence (a) to the nucleotide sequence (b). The term "loop region" refers to a portion of nucleotide sequence that takes the form of a single strand loop without self-association of nucleotide sequences in such region based on complementarity or via other means. A technique for designing a nucleotide sequence that encodes such a loop region is well-known in the art. The length of a loop region is approximately 6 bp to 15 bp, and preferably approximately 12 bp. The nucleotide sequences (c) that are preferable in the present invention are shown in SEQ ID NOs: 2, 5, and 6. It should be noted that sequences that can be employed in the present invention are not limited thereto.

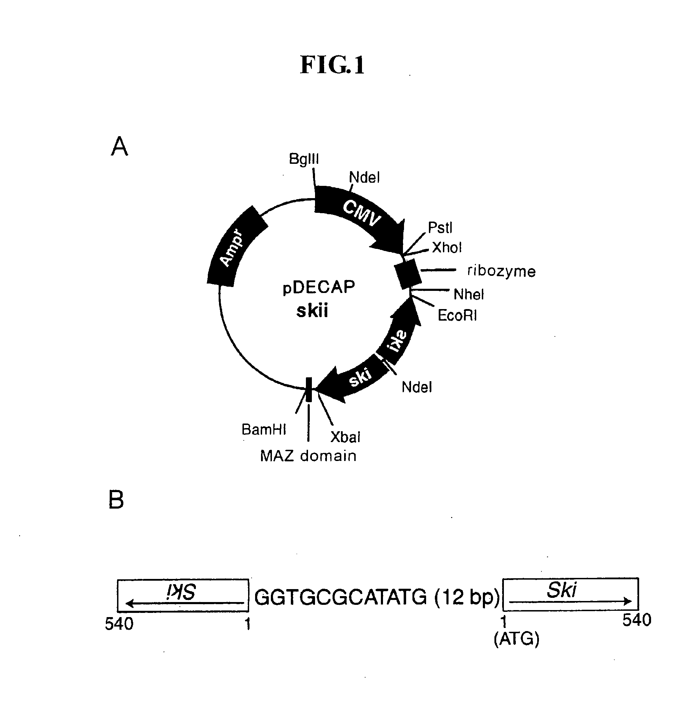

As shown in Fig. 1B, the nucleotide sequence (a) is connected to the nucleotide sequence (b) via the nucleotide sequence (c). Upon transcription of these sequences into RNA, accordingly, they form double-stranded RNA (ds-RNA) having a stem-loop structure via self-association of the nucleotide sequences (a) and (b) based on complementarity. The resulting ds-RNA induces RNA interference on the target gene.

A promoter and/or other control sequence may be operably ligated and inserted into the vector of the present invention so that the sequence of the ds-RNA region is then transcribed and ds-RNA is formed. The phrase "operably ligated and inserted into the vector of the present invention" refers to a procedure whereby a promoter and/or other control sequence are ligated and introduced into the cell or the transgenic animal to which the vector of the present invention is to be introduced so that ds-RNA for the target gene is expressed and formed under the control of the promoter and/or the other control sequence and mRNA of the endogenous target gene is disrupted and degraded. Promoters and/or control sequences that can be incorporated into the vector of the present invention are not particularly limited. Any promoter and/or a control sequence known in the art can be adequately selected in accordance with various conditions such as the type of animal used and properties of the target gene to be knocked-down. Examples thereof include a constitutive promoter, a tissue-specific promoter, a time-specific promoter, and other regulatory elements. In the present invention, a polymerase II promoter is preferably used. This is because a polymerase II promoter induces tissue-specific gene expression and thus tissue-specific expression of ds-RNA can occur in an organism.

When a Ski-knockdown animal model for disease is prepared, for example, a developmental-stage-specific promoter is preferably incorporated into the vector of the present invention since Ski-gene-associated diseases are known to develop during the developmental stage.

In the present invention, the term "developmental-stage-specific promoter" refers to a promoter that has been demonstrated to drive an expression specifically during the developmental stage. Such promoters are known in the art. Examples thereof include, but are not limited to, a cytomegalovirus (CMV) early gene promoter, an insulin gene promoter, a Lck gene promoter, a CD19 gene promoter, and a Nestin gene promoter. These promoters are known in the art, and a person skilled in the art can easily obtain the sequence information thereof.

In animal cells, ds-RNA may inhibit various types of mRNA translation upon transportation thereof from the nucleus to the cytoplasm, or it may activate ds-RNA-dependent kinase. In order to avoid such problems, a sequence that autocatalytically cleaves RNA and/or a sequence that pauses RNA polymerase may be ligated to the vector of the present invention. The term "sequence that autocatalytically cleaves RNA" refers to a sequence that can autocatalytically cleave RNA at the site of such sequence. More specifically, it refers to a sequence that autocatalytically cleaves RNA by the ribozyme activity (Huang, Y., Mo. Cell Biol. 16: 1534-1542, 1996). A person skilled in the art can easily design the nucleotide sequence of the sequence that autocatalytically cleaves RNA. An example thereof is, but is not limited to, any ribozyme sequence, such as the sequence as shown in SEQ ID NO: 3. Since the cap structure at the 5' end is critical for transportation of mRNA to the cytoplasm (McKendrick, L. *et al*., Mol. Cell Biol., 21: 3632-3641, 2001), a sequence that autocatalytically cleaves RNA is inserted immediately downstream of the transcription initiation site of ds-RNA for the target gene, and the cap structure is removed from the ds-RNA synthesized by transcription. Thus, the aforementioned problem can be avoided. Existence of the sequence that autocatalytically cleaves RNA can result in autocatalytic cleavage of the transcribed ds-RNA at the site of such sequence, and the cap structure is then removed.

The term "sequence that pauses RNA polymerase" refers to a sequence that pauses RNA polymerase on the template DNA to terminate its transcription. More specifically, it refers to a sequence to which a transcription factor binds (Yonaha, M. *et al*., Mol. Cell 3: 593-600, 1999; Yonaha, M. *et al.*, EMBO J. 19: 3770-3777, 2000). A person skilled in the art can easily design such sequence that pauses RNA polymerase. An example thereof is, but is not limited to, a sequence of a transcription factor, i.e., the MAZ domain. An example of the sequence of this MAZ domain is shown in SEQ ID NO: 4, although this example is not exclusive. A poly(A) tail at the 3' end is important in order to transport mRNA to the cytoplasm (Huang, Y., cited above), and mRNA is transported to the cytoplasm due to the existence of the poly(A) tail. In the present invention, however, it is necessary to retain the ds-RNA formed upon transcription in the nucleus and induce RNA interference. This can be realized by removing the poly(A) tail; however, a poly(A) addition signal is generally essential in order to terminate transcription. The present inventors considered that the aforementioned problem could be resolved by replacing the poly(A) addition signal on the vector with a sequence that terminates transcription (e.g., the sequence of the MAZ domain). Because of the existence of the sequence that pauses RNA polymerase, transcription of ds-RNA is terminated at the site of such sequence, and no poly(A) tail is added to the ds-RNA to be synthesized.

In order to insert the aforementioned constituents into an adequate vector, purified DNA is first cleaved with an adequate restriction enzyme, the cleaved DNA is inserted into the restriction enzyme site or multi-cloning site of an adequate vector DNA, and the resultant is then ligated to the vector. DNA (the aforementioned constituent) can be synthesized and purified by a technique known in the art.

Examples of vectors that can be used in the present invention include plasmid DNA, cosmid DNA, bacterial artificial chromosome (BAC) DNA, retrotransposon DNA, yeast artificial chromosome (YAC) DNA, and P1 phage-derived artificial chromosome (PAC) DNA. A short pUC plasmid (e.g., a plasmid of approximately 1 kbp to 3 kbp) is particularly preferable.

The thus obtained vector induces expression and formation of ds-RNA for the target gene upon introduction thereof into a cell, tissue, and/or organism, which leads to disruption and degradation of mRNA of the endogenous target gene. In this description, such vector is referred to as a pDECAP vector (Deletion of Cap structure and poly(A)).

The ds-RNA expression vector can preferably suppress tissue-specific expression of the gene product encoded by a specific endogenous gene. Accordingly, the ds-RNA expression vector of the present invention is useful for a variety of applications such as analysis of gene functions and gene therapy.

### 3. Cell into which ds-RNA expression vector has been introduced

In the present invention, a ds-RNA expression vector that expresses ds-RNA for the target gene can be introduced into a cell, thereby allowing ds-RNA for the target gene to express therein. Since mRNA of the endogenous target gene is disrupted upon formation of ds-RNA for the target gene, expression of the target gene can be suppressed.

Cells into which the ds-RNA expression vectors have been introduced are not particularly limited. Animal cells, such as cells of humans, mice, cattle, sheep, goats, pigs, rabbits, and rats, are preferable.

The ds-RNA expression vector can be introduced into cells via any means without particular limitation. For example, a method involving the use of calcium ions and electroporation can be employed.

The thus prepared cells suppress the expression of the target gene. Therefore, they are useful for analyzing gene functions and *ex vivo* suppression of the target gene expression.

### 4. Production of transgenic animal (target gene-knockdown animal)

The target gene-knockdown animal (transgenic animal) of the present invention has ds-RNA for the target gene, and mRNA of the endogenous target gene is disrupted due to the formation of ds-RNA for the target gene. This transgenic animal can be generated by introducing the ds-RNA expression vector into an animal cell and then allowing the animal cell to grow. In the thus generated transgenic animal, target gene knockdown is realized via disruption and degradation of mRNA of the endogenous target gene due to the presence of ds-RNA. In the present invention, the term "endogenous" refers to the natural presence of a gene in the cell and/or the organism to which the vector of the present invention is to be introduced. It also refers to the presence therein of a gene since before the vector was introduced. The term "knockdown" used herein refers to reduced expression of a gene product (e.g., a protein). Animals to which the ds-RNA expression vector of the present invention is to be introduced are not particularly limited as long as they are mammals. Examples thereof include mice, cattle, sheep, goats, pigs, rabbits, and rats. In the present invention, mice, rats, and the like are preferable since they are easy to handle as animal models of disease.

The vector of the present invention is preferably introduced into an animal cell by injection into the fertilized egg of the target animal. As a result, the sequence encoding the ds-RNA for the target gene and a control sequence are incorporated into the fertilized egg cell, and the sequence encoding the ds-RNA and a control sequence are copied in cells during division of the egg.

Genes can be introduced into animal cells using techniques such as the method of introduction into ES cells and the method of introducing a cell nucleus introduced into the culture cells via nuclear transplantation into fertilized eggs, in addition to microinjection into fertilized eggs. DNA of the vector of the present invention can be introduced into ES cells or other culture cells by electroporation, lipofection, or other means and can be subjected to positive selection with neomycin, promycin, or the like. Thus, the cells of interest may be obtained. ES cells are injected into nascent embryos or 8-cell-stage embryos using capillaries or the like. Nuclear transplantation is carried out by injecting the cells into which DNA has been introduced into the fertilized eggs from which the nucleus has been removed, and cell fusion is carried out by electrical stimulation.

Thereafter, nascent embryos or 8-cell-stage embryos are directly transplanted into the ovarian duct of a foster mother. Alternatively, nascent embryos or 8-cell-stage embryos are cultured for a day and embryos that have developed to blastocysts are then transplanted into the uterus of a foster mother. The foster mother is raised and made to give birth to offspring to obtain a transgenic animal (chimeric animal), which is an offspring into which the target ds-RNA has been introduced. In order to confirm the expression of a desired ds-RNA in the animal, a part of the animal body (for example, a caudal tip) is collected, DNA in the somatic cell is extracted, and the presence of the introduced sequence is confirmed by PCR or Southern blotting. The animal, once the presence of the sequence introduced therein has been confirmed, is determined to be the first generation, and 50% of the ds-RNA is then genetically transmitted to its offspring (F1). More specifically, mating between a transgenic animal and a normal animal can produce a heterozygous animal (F1), and mating between heterozygotes can produce a homozygous animal (F2). Further, the offspring of the target gene-knockdown animal can be obtained by repeating mating between animals of the same species.

Thus, a transgenic animal with specific target gene knockdown is useful as an animal model for disease or as an animal for genetic analysis as described below.

### 5. Animal model for disease

If the target gene of the transgenic animal produced in the manner described above is knocked down (mutated), this transgenic animal can be utilized as an animal model exhibiting several types of diseases (an animal model for disease). For example, a transgenic Ski-knockdown animal may exhibit neural tube closure defects, malformation of the iris in the eye, or hemorrhage in the head. Hemorrhage in the head was first found by the present inventors. Accordingly, the animal model for disease according to the present invention is useful for research of such diseases, development of therapy techniques for such diseases, and other purposes.

### Examples

The present invention is hereafter described in greater detail with reference to the examples, although the technical scope of the present invention is not limited thereto.

### [Example 1] Construction of Ski double-stranded RNA (ds-RNA) expression vector

A plasmid that allows the expression of hairpin RNA (i.e., RNA comprising a self-complementary stem loop) was constructed as follows: as shown in Fig. 1A, a 540 bp 5'-region (SEQ ID NO: 1) in coding region of the Ski gene was inserted into the pDECAP plasmid (shown below), and a sequence complementary to the aforementioned 540-bp sequence was inserted downstream thereof as an inverted repeat separated by a 12-bp spacer (the loop region) as shown in SEQ ID NO: 2. The resultant was designated as pDECAP-Ski.

Sequence of the spacer: 5'-GGTGCGCATATG-3' (SEQ ID NO: 2)

pDECAP comprises pcDNA3 (Invitrogen) having a CMV promoter, a ribozyme cassette cutting off the cap structure at the 5' end of RNA transcribed as a sequence that autocatalytically cleaves RNA (SEQ ID NO: 3; Huang, Y. and Carmichael, GC, 1996, Mol. Cell Biol. 16: 1534-1542), and a MAZ domain as a sequence for pausing RNA polymerase II transcription (SEQ ID NO: 4; Yonaha, M. and Proudfoot, NJ, 1999, Mol. Cell 3: 593-600). This construct does not have any poly(A) addition signal.

mRNA transcribed from this expression vector forms a stem-loop structure at the 12-bp spacer portion thereof and becomes ds-RNA having the sequence of Ski mRNA.

In the same manner as described above, the first 518 bp of the firefly luciferase-gene-encoding region or the sequence consisting of nucleotides 443-944 of the β-galactosidase gene are separated by a 12-bp spacer (SEQ ID NO: 5 or 6, respectively) from a sequence complementary thereto as an inverted repeat thereof. Thus, a firefly luciferase-targeted ds-RNA expression vector (pDECAP-Luc) and a β-galactosidase-targeted ds-RNA expression vector (pDECAP-β-gal) were constructed.

The pDECAP plasmids containing the inverted repeats, thus constructed, were amplified in the *E. coli* Sure 2 strain (Stratagene), which allows the accurate replication of DNA containing inverted repeats, and purified using the EndoFree Plasmid Maxi Kit (Qiagen).

The pDECAP-LucS plasmid expressing sense RNA of the firefly luciferase gene, the pDECAP-LucAS plasmid expressing antisense RNA of the firefly luciferase gene, a common pCMV-LucS plasmid expressing RNA of the firefly luciferase gene containing the 5'-cap structure and a poly(A) tail, and the pCMV-gali plasmid containing a 5'-cap structure and a poly(A) tail and expressing ds-RNA for β-galactosidase were constructed.

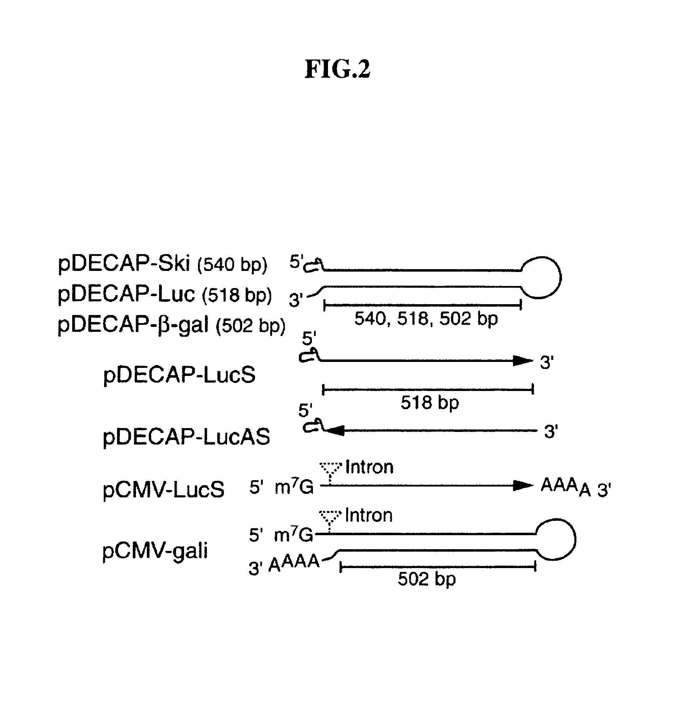

The predicted secondary structures of RNAs transcribed from the plasmids thus constructed are shown in Fig. 2. pDECAP-Ski, pDECAP-Luc, and pDECAP-β-gal encode a 540- to 502-bp hairpin ds-RNA for Ski, firefly luciferase, and β-galactosidase, respectively, which lack the 5'-cap structure and a poly(A) tail. Sense and antisense single-stranded RNA of the luciferase gene were transcribed from pDECAP-LucS and pDECAP-LucAS, respectively. pCMV-gali encodes the same hairpin ds-RNA as pDECAP-β-gal, except that it contains the 5'-cap structure, a splicing intron, and a poly(A) tail.

### [Example 2] Reduced Ski expression level caused by Ski ds-RNA expression vector

In this example, the effect of the Ski ds-RNA expression vector on the Ski protein level in cultured cells was examined.

### (1) Southern blotting

293 T cells from human kidney were cultured in DMEM (Invitrogen) containing 10% FBS and an antibiotic (penicillin/streptomycin; Invitrogen). The pDECAP-Ski plasmid prepared in Example 1 and 0.05 µg of TK-Luc (a firefly luciferase expression vector; Promega) as the internal control were transfected into 4 x 10⁵ 293T cells using Lipofectamine (GibcoBRL). Cells were lysed in Passive Lysis Buffer (Promega) 42 hours later, and aliquots of the cell extracts were subjected to luciferase assay (Promega) to evaluate the transfection efficiency.

The remaining cell pellets were resuspended in a RIPA lysis buffer (1% Triton X-100, 1% sodium deoxycholate, 0.1% SDS, 0.15M NaCl, 0.01M sodium phosphate (pH 7.2), and 1% trasylol), and the resultant was adjusted in accordance with the luciferase assay results. A mixture of anti-Ski monoclonal antibodies 1-1, 9-1, 11-1, and 16-1 (Nomura *et al*., 1999, *supra*) was used for immunoblotting (Western blotting) in accordance with the method of Shinagawa *et al*. (Shinagawa, T, *et al*., 2000, EMBO J., 19: 2280-2291).

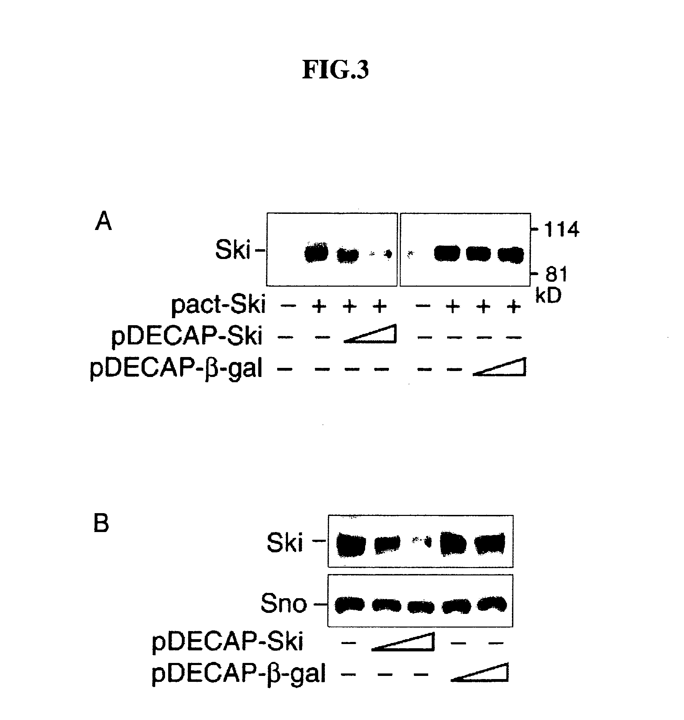

A band of approximately 90 kD for Ski protein was detected by Western blotting. A mouse Ski expression vector (pact-ski; Nomura, T. *et al*., Genes & Development 13: 412-423, 1999) and various amounts of Ski ds-RNA expression vectors (pDECAP-Ski) were introduced to perform Western blotting in the same manner. As a result, the Ski protein level was found to decrease depending on the amount of ds-RNA (Fig. 3A).

As a negative control, the pDECAP-β-gal plasmid prepared in Example 1 was also introduced into the 293T cell together with the mouse Ski expression vector (pact-Ski) to analyze the Ski expression level. In this case, the Ski protein level was not decreased (Fig. 3A). Accordingly, Ski ds-RNA was found to decrease the Ski expression level specifically in cultured animal cells.

The pDECAP-Ski and pDECAP-β-gal plasmids were transfected into the 293T cells, and endogenous Ski and Sno proteins were detected by immunoblotting. When the pDECAP-Ski vector was independently transfected into the 293T cell, the level of endogenous Ski protein decreased (Fig. 3B). The level of the endogenous Sno protein having approximately 60% homology to the target sequence of pDECAP-Ski did not change.

### (2) Northern blotting

293T cells were transfected with 0.8 µg of various types of expression vectors shown in Fig. 2 (pDECAP-Ski, pDECAP-Luc, pDECAP-β-gal, pDECAP-LucS, pDECAP-LucAS, pCMV-LucS, or pCMV-gali). Cytoplasmic and nuclear RNAs were extracted therefrom 48 hours later. The subcellular distribution of RNAs was examined by Northern blotting using a digoxigenin (DIG)-labeled LucS DNA probe.

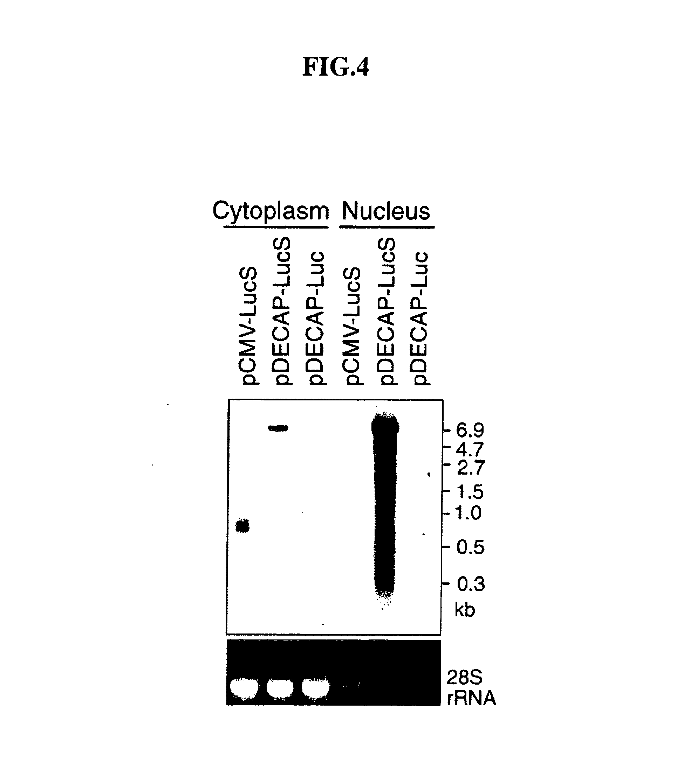

The results are shown in Fig. 4. The upper photograph in Fig. 4 shows the results of Northern blotting, and the lower photograph shows the control in order to demonstrate that the RNA levels are the same in all lanes. While most of the luciferase sense RNAs transcribed from pDECAP-LucS were in the nucleus, the luciferase sense RNA transcribed from pCMV-LucS was in the cytoplasm. The size of the luciferase transcripts from pDECAP-LucS corresponds to that of a plasmid. This suggests that the long RNAs were transcribed by a read-through mechanism due to a lack of the poly(A) addition signal. The long RNA transcribed from pDECAP-Luc was detected neither in the cytoplasm nor in the nucleus by Northern blotting. This suggests that it was immediately digested into small RNAs.

### (3) Interferon response

To investigate whether Ski mRNA expressed from pDECAP-Ski induces an interferon response, the phosphorylation status of eIF2α, which is a substrate of the ds-RNA-activated PKR protein kinase, was examined. 293T cells were cotransfected with pDECAP empty plasmid or pDECAP-Ski plasmid (0.6 µg) and pCMV-β-gal plasmid (0.3 µg) as an internal control. The cells were harvested 48 hours later and subjected to immunoblot analysis using a phospho-specific antibody against eIF2α phosphorylated on Ser 51 (eIF2α-P). The same membrane was reprobed with antibodies against total eIF2α and β-galactosidase. The lysate of 293T cells treated with poly(I)·poly(C) (100 µg/ml) for 24 hours was used as a positive control.

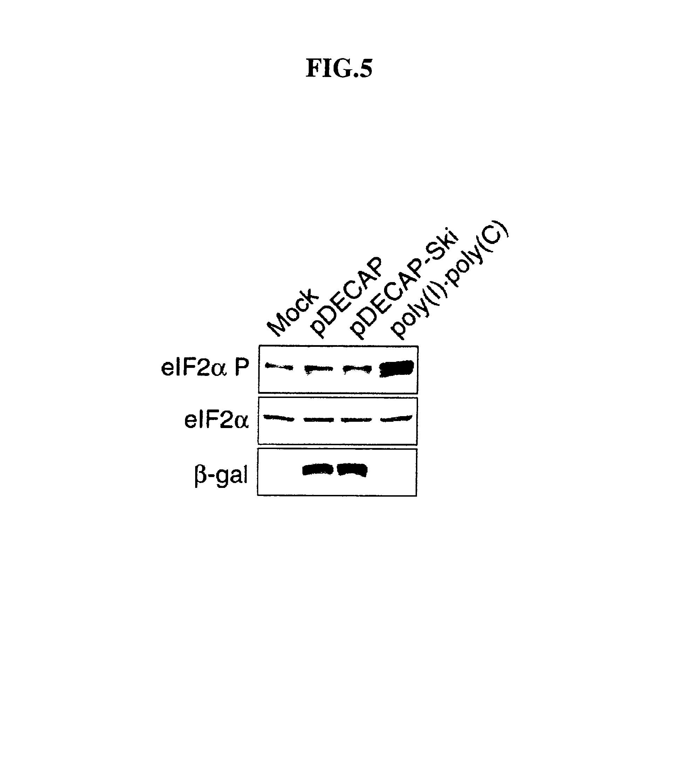

The results are shown in Fig. 5. The levels of phosphorylated eIF2α were not enhanced by transfection of 293T cells with pDECAP-Ski, whereas the phosphorylation of eIF2α was stimulated in the control cells treated with poly(I)·poly(C) (Fig. 5). This indicates that interferon response was not induced in the cells into which pDECAP-Ski had been introduced.

### (4) RT-PCR assay

To determine the level of endogenous Ski mRNA, the reverse transcriptase PCR (RT-PCR) was carried out. More specifically, 1.5 µg each of pDECAP empty plasmid, pDECAP-Ski plasmid, and pDECAP-β-gal plasmid were transfected into 293T cells together with enhanced green fluorescent protein (EGFP) expression plasmid (pMSCV-ir-EGFP). EGFP-positive cells were collected by sorting and then treated with actinomycin D. Total RNA was isolated at various time points and the level of Ski mRNA was analyzed by RT-PCR. The RT-PCR products were separated on 2% agarose gels and visualized by ethidium bromide staining.

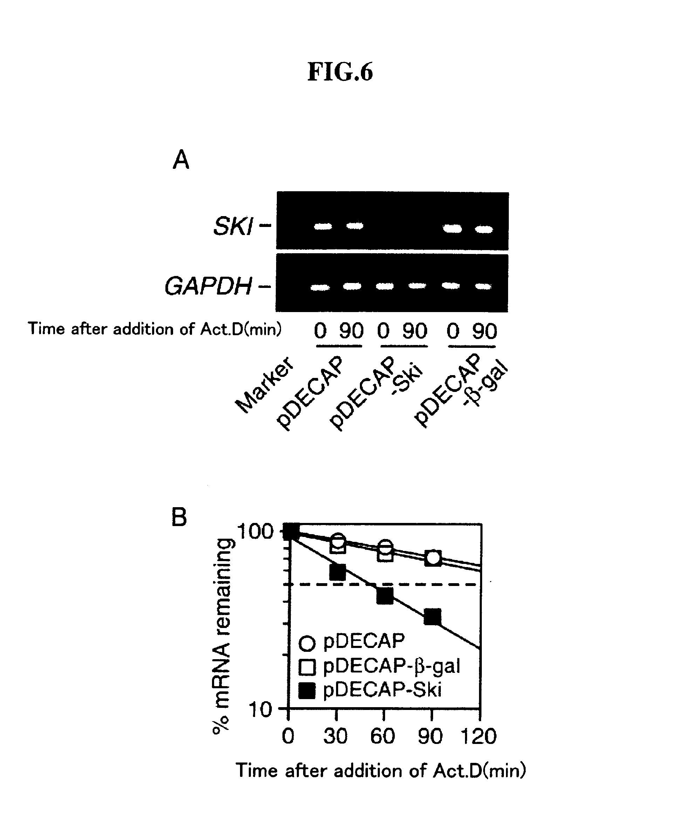

The results are shown in Fig. 6A and Fig. 6B. Fig. 6A shows the results of RT-PCR demonstrating the degradation of Ski mRNA by the Ski ds-RNA expression vector. Fig. 6B shows the results of RT-PCR that analyzed the degradation of Ski mRNA by the Ski ds-RNA expression vector over time.

RT-PCR demonstrated that the levels of endogenous Ski mRNA were also reduced by transfection with pDECAP-Ski (Fig. 6B). To confirm that the decreased Ski mRNA levels were due to the degradation of Ski mRNA, Ski mRNA levels at various time points were measured after treatment of the transfected cells with an inhibitor of transcription, i.e., actinomycin D (Fig. 6B). In the cells transfected with the pDECAP-Ski plasmid, the half-life of endogenous Ski mRNA was 0.9 hours, whereas it was 2.7 and 3.1 hours in cells transfected with the pDECAP plasmid expressing β-galactosidase ds-RNA (pDECAP-β-gal) and the empty plasmid, respectively. This indicates that Ski ds-RNA expressed from the pDECAP vector stimulated the degradation of Ski mRNA.

### [Example 3] Luciferase assay of mouse embryonic fibroblast cells

Mouse embryonic fibroblast cells were cultured in Dulbecco's Modified Eagle medium (DMEM) containing 10% fetal bovine serum (FBS) and antibiotics. Cells (0.5 x 10⁵ cells) were transferred to 12-well plates (1 ml per well) and transfected with mixtures of the firefly luciferase (Pp-luc) expression plasmids (0.1 µg, TK-βRex2-Luc, containing the thymidine kinase promoter and β-retinoic acid receptor-binding sites), the plasmids constructed in Example 1 (0.4 µg, pDECAP-ski, pDECAP-Luc, pDECAP-β-gal, pCMV-gali, pDECAP-LucS, and pDECAP-LucAS), and the *Renilla reniformis* luciferase (Rr-luc) expression plasmid (0.02 µg, pRL-CMV) using Lipofectamine (Invitrogen) according to the manufacturer's instructions. All-*trans* retinoic acid (1 µM) was added thereto 24 hours later to induce expression of the firefly luciferase gene (Pp-luc) and cells were cultured for an additional 36 hours. Luciferase expression was subsequently monitored with the dual luciferase assay (Promega).

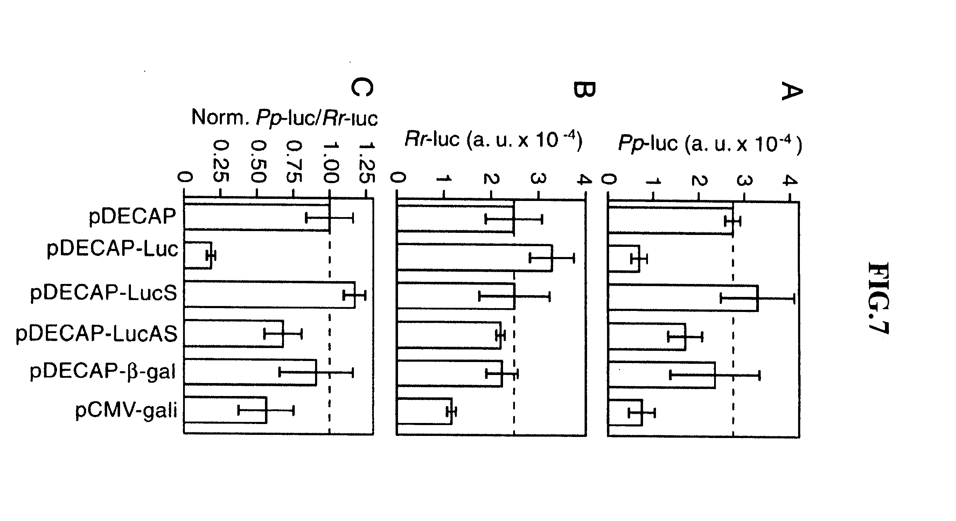

The results are shown in Fig. 7A to Fig. 7C. Fig. 7A shows firefly luciferase (Pp-luc) activity plotted in arbitrary luminescence units (a.u.). Fig. 7B shows *Renilla reniformis* luciferase (Rr-luc) activity plotted in arbitrary luminescence units. Fig. 7C shows ratios of the normalized target to the control luciferase.

Firefly luciferase (Pp-luc) levels were reduced about fivefold on cotransfection with the pDECAP plasmid (pDECAP-Luc) expressing firefly luciferase ds-RNA (Fig. 7A). In contrast, *Renilla* luciferase (Rr-luc) activity was not affected (Fig. 7B). Cotransfection of the pDECAP plasmid (pDECAP-LucS) expressing sense RNA for the same region of the firefly luciferase gene did not affect the firefly luciferase activity. In contrast, cotransfection of the pDECAP plasmid (pDECAP-LusAS) expressing antisense RNA for the same region had a slight effect (about a 25% reduction). This indicates that the structure of the ds-RNA is critical for effectively inhibiting firefly luciferase activity. Further, coexpression of 502 bp ds-RNA for the β-gatactosidase gene from the pDECAP plasmid did not affect the firefly or *Renilla reniformis* luciferase activity. In the case of coexpression of ds-RNA for β-galactosidase containing a 5'-cap structure, splicing sites, and a poly(A) tail from another plasmid (pCMV-gali, which had the CMV promoter but lacked both ribozyme and MAZ sites), the activities of both luciferases were decreased. This indicates that the removal of the cap structure and the poly(A) is required to suppress nonspecific inhibition of translation.

### [Example 4] Generation of transgenic mouse into which Ski ds-RNA expression vector has been introduced

### (1) Southern blotting

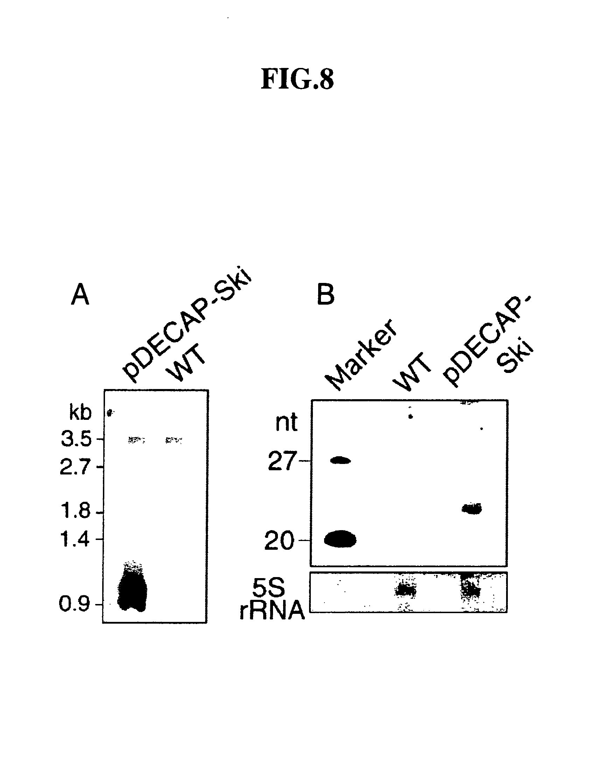

A 2.2 kb BgIII-BamHI fragment in pDECAP-Ski or pDECAP-gali was cleaved out of the background sequence, purified, and then injected into a mouse fertilized egg. The mouse fertilized egg was derived from mating between a male C57BL/6N mouse and a female C57BL/6N or (C57BL/6JxDBA) F1 mouse (obtained from CLEA). To examine the presence of the transgenes and the copy number thereof, the embryo was analyzed by Southern blot analysis utilizing the first 540 bp fragment of the Ski-coding region as a probe. Genomic DNA used for Southern blot analysis was extracted from the extraembryonic tissue. The results are shown in Fig. 8A. In Fig. 8A, the left lane shows the results of analyzing the pDECAP-Ski transgenic mouse and the right lane shows those for the wild-type mouse (WT). This demonstrates that the transgenic mouse actually had the Ski ds-RNA expression vector. The transgene copy number was deduced to be 2 to 12.

### (2) Northern blotting

To examine whether the short RNA was generated in the transgenic mouse embryo, Northern blotting was carried out using the Ski sense-strand probe.

Nuclear RNA (5 µg) was extracted from the head of a transgenic mouse embryo and then analyzed by Northern blotting. The membrane was probed with a DIG-labeled sense Ski probe corresponding to the target sequence. Fig. 8B (lower part) shows 5S rRNA visualized by staining with methylene blue on the membrane. The results indicate that the small RNA of about 21 or 22 nucleotides was detected in the pDECAP-Ski transgenic mouse embryo but was not detected in the wiid-type embryo (Fig. 8B).

### (3) Real time RT-PCR

To analyze the mouse embryos, RNA was isolated from the limb bud of an embryo using ISOGEN (Nippon Gene Co., Ltd.). Expression of transgenes was assayed via real-time RT-PCR using the primer pair shown below and the dual-labeled fluorescent probe at the 3' end of the ribozyme cassette having the following sequence.
(Primer pair) CCGCCTGATGAGTCCGTGAG (SEQ ID NO: 7) ATCGAAGTCATGGTGGCTA (SEQ ID NO: 8) (Probe)

GACGAAACATGCATAGGC (SEQ ID NO: 9)

The results of RT-PCR are shown in Table 1. In Table 1, the expression levels of the transgenes are represented by relative values when the embryo No. 257 is determined to be 1.00.

**Table 1**

| Genotype | Embryo (No.) | Expression level of transgene | Neural tube defects |
|---|---|---|---|
| skii | 256 | 0.015 | - |
| | 257 | 1.00 | + |
| | 259 | <0.002 | - |
| | 262 | 4.58 | + |
| gali | 233 | 9.15 | - |
| | 268 | <0.002 | - |

### [Example 5] Murine model of disease

### (1) Neural tube defects

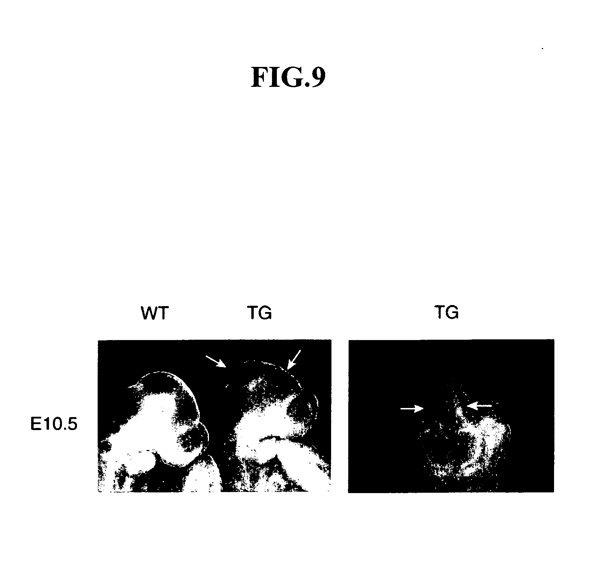

It has already been reported that Ski mutant mice have neural tube closure defects (Berk, M. *et al*, Genes Dev. 11: 2029-2039, 1997). The present inventors analyzed the Ski ds-RNA-expressing transgenic mice at E10.5 and confirmed that such transgenic mice exhibited similar neural tube closure defects (Fig. 9). As indicated by an arrow in Fig. 9, the neural tubes of the transgenic (TG) mice were not closed. Among 13 transgenic embryos, 5 (38.5%) exhibited neural tube closure defects (see Table 1). The β-galactosidase ds-RNA-expressing transgenic mice (9 mice) prepared as negative controls did not exhibit neural tube closure defects.

### (2) Defects in eye formation

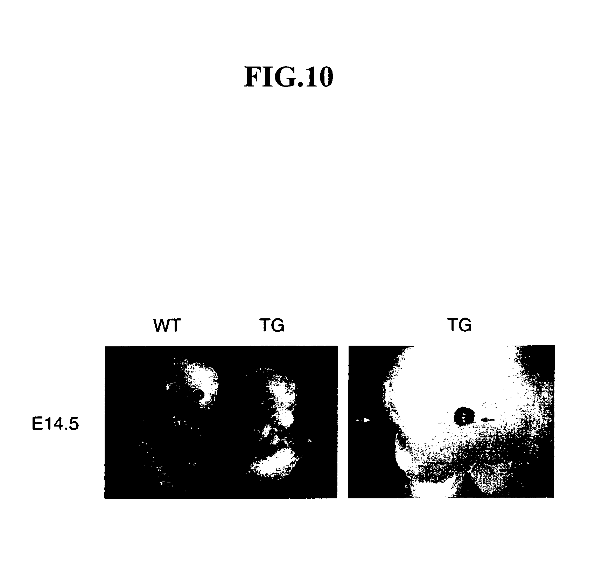

It has already been reported that Ski mutant mice exhibit malformations of the irises in their eyes (Colmenares, C. *et al*., Nature Genet. 30: 106-109, 2002). The present inventors analyzed the Ski ds-RNA-expressing transgenic mice at E10.5 and confirmed that such transgenic mice developed malformations of the irises in their eyes (Fig. 10). As indicated by an arrow in Fig. 10, transgenic (TG) mice lack part of the irises in their eyes. Each of two transgenic embryos (100%) exhibited malformations of the irises in the eyes. The β-galactosidase ds-RNA-expressing transgenic mice (9 mice) prepared as negative controls did not exhibit malformations of the irises in the eyes.

### (3) Hemorrhage in the head

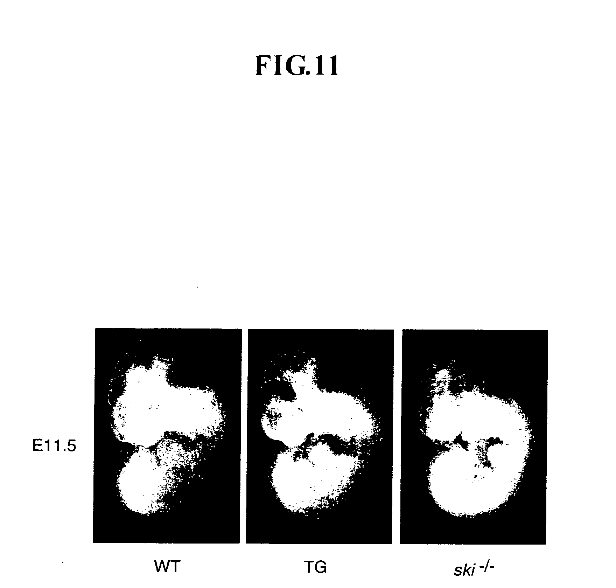

The present inventors observed that the Ski ds-RNA-expressing transgenic mice developed hemorrhages in their heads (Fig. 11). In Fig. 11, the transgenic (TG) mice and the Ski mutant mice (ski^{-/-}) exhibit hemorrhages in their heads. This demonstrates that the Ski mutant mice can be employed as murine models for hemorrhage in the head.

Based on the results shown in (1) to (3) above, the Ski ds-RNA transgenic mice exhibit defects and hemorrhage in the head, as with the Ski mutant mice. In general, the effects of transgenes are observed in 20% to 80% of transgenic mice individuals. This is because the transgene may not be expressed due to the position of the gene integrated (position effects). Neural tube closure defects were observed in 38.5% of the Ski ds-RNA transgenic mice constructed by the present inventors. This rate is reasonable based on past reports concerning transgenic mice.

### [Example 6] Introduction of ds-RNA and frequency of phenotype expression

Transgenic mouse embryos containing the β-galactosidase expression vector driven by the CMV promoter (pCMV-β-gal) were prepared in order to examine whether or not the CMV promoter exhibits activity in various regions of mouse embryos.

In two of six embryos of pCMV-β-gal transgenic mice, the β-galactosidase was expressed in almost all tissues, including the neuroepithelial cells, at E9-E12. This frequency (33.3%, 2/6) is similar to that of the embryos of pDECAP-Ski transgenic mice exhibiting abnormalities (Example 5) and is within the average frequency (10% to 40%) of transgenic mice showing the specific phenotype caused by the injected DNA. β-galactosidase expression was frequently observed in various tissues, including the eyes, of the embryos of pCMV-β-gal transgenic mice at E15. This is consistent with the case where β-galactosidase expression was frequently observed in embryos of pDECAP-Ski transgenic mice exhibiting eye defects at E14-E16.

As control experiments, transgenic mice were prepared by injecting a fragment containing the expression unit of 502-nt β-galactosidase ds-RNA prepared from the pDECAP-β-gal. No abnormality was observed in any of the nine mouse embryos analyzed (Table 2). This indicates that the abnormalities observed in the pDECAP-Ski transgenic mice were specifically caused by Ski ds-RNA, but were not caused by long ds-RNA in a non-specific manner. Although the typical abnormalities of Ski-deficient mice such as neural tube defects and eye defects were observed in pDECAP-Ski transgenic mice, other defects such as postaxial polydactyly of Ski-deficient mice were not observed therein. This may be because the CMV promoter was not sufficiently active in some tissues.

**Table 2**

| DNA injected | Stage of analyzed embryos | Number of transgenic embryos obtained | Number of embryos exhibiting phenotypes/number of embryos analyzed | | |
|---|---|---|---|---|---|
| | | | Neural tube defect | Eye defect | β-gal expression |
| pDECAP-Ski | E9-E12 | 13 | 5/13 (38.5%) | 2/2 (100%)^{a} | ND |
| | E14-E16 E9-E11 | 2 | 0/2 (0%) | 2/2 (100%) | ND |
| pDECAP-β-gal | E9-E12 | 9 | 0/9 (0%) | ND | ND |
| pCMV-β-gal | E15 | 6 | 0/6 (0%) | ND | 2/6 (33.3%) |
| | | 1 | 0/1 (0%) | 0/1 (0%) | 1/1 (100%) |

| | | | | | |
|---|---|---|---|---|---|
| a: Ski expression in lens vesicle of E 10.5 embryos | | | | | |
| (ND): Not determined | | | | | |

### [Example 7] Histological analysis of transgenic mice

Hematoxylin-eosin staining of tissue, *in situ* hybridization using RNA, and Tunel assay were performed in order to examine the influences of endogenous Ski gene expression and RNAi on transgenic mice.

Hematoxylin-eosin staining was performed by placing samples on glass slides and immersing the glass slides in each solution in the following manner:
1) a hematoxylin solution at room temperature for 3 minutes;
2) rinsing under running water;
3) 1% eosin at room temperature for 7 minutes;
4) 50% ethanol at room temperature for 2 seconds 7 times;
5) 70% ethanol at room temperature for 2 seconds 7 times;
6) 90% ethanol at room temperature for 2 seconds 7 times;
7) 95% ethanol at room temperature for 2 seconds 7 times;
8) 100% ethanol at room temperature for 15 minutes;
9) 100% ethanol at room temperature for 15 minutes;
10) 100% ethanol at room temperature for 15 minutes;
11) xylene at room temperature for 15 minutes;
12) xylene at room temperature for 15 minutes;
13) xylene at room temperature for 15 minutes; after which
14) xylene was evaporated to dryness, mounting medium was mounted on glass slides, and coverslips were placed thereon.

*In situ* hybridization was performed in accordance with the method of Butler, K. *et al.* (Methods, vol. 23, pp. 303-312, 2001). For detection of Ski mRNA, the 3'-half of the Ski gene was used as the probe. More specifically, a plasmid spanning nucleotides 754-2223 of the mouse c-Ski cDNA was linearized with BamHI and transcribed with T7 RNA polymerase to prepare the c-Ski 3' probe to be used. The ODC probe for Odc gene detection has been already reported by Nomura, T. *et al.* (Genes & Dev., 1999, vol. 13, pp. 412-423).

The TUNEL assay was employed to study apoptosis using a commercially available apoptosis kit (*in situ* cell death detection kit, Roche) according to the manufacturer's instructions.

The results of the above experiments are shown in Figs. 12A to 12G. In Figs. 12A to 12G, "a" represents a wild-type mouse, "b" represents a pDECAP-β-gal transgenic mouse, "c" represents a pDECAP-Ski transgenic mouse, and "d" represents a Ski-deficient mouse (Ski^{-/-}). Fig. 12A shows hematoxylin-eosin staining of head sections. Fig. 12B shows Ski mRNA expression in midbrains and mesenchymes as detected by RNA *in situ* hybridization. Fig. 12C shows Odc mRNA expression in midbrains and mesenchymes as detected by RNA *in situ* hybridization. Fig. 12D shows apoptotic cells assessed by the TUNEL assay on the neuroepitheliums of midbrains and mesenchymes. Fig. 12E shows hematoxylin-eosin staining of eyes. Fig. 12F shows Ski mRNA expression in eyes detected by *in situ* hybridization. Fig. 12G shows the results of the TUNEL assay on eyes. In Fig. 12A and 12E, "MB" represents the midbrain, "II" represents the second ventricle, "III" represents the third ventricle, "O" represents the optic cup, and "L" represents the lens vesicle.

In the neural epithelial cells of the pDECAP-Ski transgenic mice and the Ski-deficient mice, which were generated by homologous recombination in ES cells, Ski mRNA was not detected (Fig. 12B: c and d). In contrast, Ski mRNA was expressed in the neural epithelial cells of pDECAP-β-gal ds-RNA transgenic mice and wild-type mice (Fig. 12B: a and b). These results were confirmed by RT-PCR (data not shown).

The neural tube closure defects of Ski-deficient mice were caused by the ectopic expression of the ornithine decarboxylase (Odc) gene followed by apoptosis in neural epithelial cells. This is because Ski is required for Mad-mediated transcriptional repression of the Odc gene (Nomura, T. *et al*., Genes & Dev., 1999, vol. 13, pp. 412-423). Similar ectopic expression of the Odc gene was observed in neural epithelial cells of embryos of pDECAP-Ski transgenic mice (Fig. 12C: c). Furthermore, ectopic apoptosis was observed in regions where the levels of Odc expression were high (Fig. 12D: c and d). These results indicate that the neural tube closure defects observed in pDECAP-Ski transgenic mice are caused by the same mechanism that operates in Ski-deficient mice generated by homologous recombination in ES cells.

During eye development, different retinal cell types, including retinal ganglion cells, cone photoreceptors, amacrine cells, and rod photoreceptors, are generated from the retinal progenitor cells in a fixed chronological sequence (Young, R. W., Anat. Rec., 1985, vol. 212, pp.199-205). In embryos of wile-type mice, Ski mRNA was detected in the lens vesicle, optic cup, and surface epithelium at E10.5 (Fig. 12F: a). In embryos of pDECAP-Ski transgenic mice and Ski-deficient mice, Ski mRNA levels in these regions were dramatically reduced (Fig. 12F: c and d). Ectopic apoptosis was observed in the lens vesicle and the surface epithelium of pDECAP-Ski transgenic mice and Ski-deficient mice, which was consistent with the above observations (Fig. 12G: c and d). However, ectopic apoptosis was not observed in embryos of wild-type mice (Fig. 12G: a). Ectopic apoptosis of the optic cup was observed in the Ski-deficient mouse embryos but was not apparent in embryos of pDECAP-Ski transgenic mice. This suggests that low levels of Ski mRNA may remain in this region.

All publications, patents, and patent applications cited herein are incorporated herein by reference in their entirety.

### Industrial Applicability

The present invention provides a target gene-knockdown transgenic animal and a ds-RNA expression vector therefor. The transgenic animal according to the present invention can be easily and rapidly produced with the use of a ds-RNA expression vector that induces RNA interference. The Ski knockdown transgenic animal according to the present invention can be utilized as, for example, an animal model for exhibiting several types of diseases. Further, the ds-RNA expression vector according to the present invention is constructed to be capable of efficiently inducing RNA interference. Thus, it can express ds-RNA for the target gene and suppress the expression of the target gene without being affected by position effects.

### Free text of sequence listing

SEQ ID NOs: 2 to 9: synthetic oligonucleotides

## Claims

1. A double-stranded RNA (ds-RNA) expression vector that comprises the following sequences (a) to (c):
(a) the following nucleotide sequence (a-1) or (a-2):
(a-1) a nucleotide sequence encoding all or a part of the target gene; or
(a-2) a nucleotide sequence encoding DNA that hybridizes under stringent conditions to DNA consisting of a sequence complementary to the nucleotide sequence (a-1);
(b) a nucleotide sequence complementary to the nucleotide sequence (a) and an inverted repeat thereof; and
(c) a sequence encoding a loop region and connecting the nucleotide sequence (a) to the nucleotide sequence (b),
wherein the sequences are transcribed into RNA and thereby forming ds-RNA having a stem-loop structure.

2. The ds-RNA expression vector according to claim 1, which further comprises a polymerase II promoter.

3. The ds-RNA expression vector according to claim 1, which further comprises a developmental-stage-specific promoter.

4. The ds-RNA expression vector according to claim 2 or 3, wherein the polymerase II promoter or developmental-stage-specific promoter is a cytomegalovirus (CMV) early gene promoter.

5. The ds-RNA expression vector according to any one of claims 1 to 4, which further comprises a sequence that autocatalytically cleaves RNA located upstream of the nucleotide sequences (a) to (c).

6. The ds-RNA expression vector according to claim 5, wherein the sequence that autocatalytically cleaves RNA is a ribozyme site.

7. The ds-RNA expression vector according to any one of claims 1 to 5, which further comprises a sequence that pauses RNA polymerase located downstream of the nucleotide sequences (a) to (c).

8. The ds-RNA expression vector according to claim 7, wherein the sequence that pauses RNA polymerase is a sequence of the MAZ domain.

9. The ds-RNA expression vector according to any one of claims 1 to 8, wherein the nucleotide sequence (c) is as shown in SEQ ID NO: 2, 5, or 6.

10. The ds-RNA expression vector according to any one of claims 1 to 9, wherein the target gene is a disease-associated gene.

11. The ds-RNA expression vector according to any one of claims 1 to 9, wherein the target gene is the Ski gene.

12. The ds-RNA expression vector according to claim 11, wherein a part of the target gene is a 540 bp 5'-region of the Ski gene.

13. A target gene-knockdown animal, in which a ds-RNA for the target gene is expressed.

14. The animal according to claim 13, in which the ds-RNA for the target gene is tissue-specifically expressed.

15. The animal according to claim 13 or 14, which is a transgenic animal having a ds-RNA expression vector introduced therein and expressing ds-RNA for the target gene, or progeny thereof.

16. The animal according to claim 15, wherein the ds-RNA expression vector is any one of those according to claims 1 to 12.

17. The animal according to any one of claims 13 to 16, wherein the target gene is a disease-associated gene, and the animal is an animal model for disease.

18. The animal according to claim 17, wherein the target gene is the Ski gene, and the disease is selected from the group consisting of neural tube closure defect, malformation of the iris, and hemorrhage in the head.

19. The animal according to any one of claims 13 to 18, wherein the animal is a mouse.

20. A method for producing a target gene-knockdown animal, wherein a ds-RNA expression vector capable of expressing ds-RNA for the target gene is introduced to form ds-RNA for the target gene.

21. The method according to claim 20, wherein the ds-RNA expression vector is any one of those according to claims 1 to 12.

22. The method according to claim 20 or 21, wherein the target gene is a disease-associated gene, and the animal is an animal model for disease.

23. The method according to claim 22, wherein the target gene is the Ski gene, and the disease is selected from the group consisting of neural tube closure defect, malformation of the iris, and hemorrhage in the head.

24. The method according to any one of claims 20 to 23, wherein the animal is a mouse.

25. An animal cell having the ds-RNA expression vectors according to any one of claims 1 to 12 introduced therein.


SEQUENCE LISTING

<110> Oriental Yeast Co.,Ltd
      RIKEN

<120> A process for producing a knock-down animal by introduction of
      a vector expressing a double-stranded RNA

<130> PH-1885-PCT

<150> JP 2002-10-29
<151> 2002-10-29

<160> 9

<170> PatentIn version 3.1

<210> 1
<211> 540
<212> DNA
<213> Mus musculus

<400> 1
atggaagctg cagcggccgg ccggggcggc ttccagcagc ccgggctgca gaagacgctg      60

gagcagttcc acctgagctc catgagctcg ctgggcggcc cggccgtttc tcggcgcgct     120

gggcaggagg cctacaagaa ggagagcgcc aaggaggcgg gtgcggccac agtgccggcg     180

ccggtgccca ctgccgcgga gccgccgccc gtgctgcacc tgcctgccat ccagccgccg     240

ccgcccgtgc ttcccgggcc cttcttcatg ccgtcggacc gctccaccga gcgctgtgag     300

accgtgctgg aaggggagac catctcttgt ttcgtggtgg gaggtgagaa gcgtctgtgc     360

ctgccgcaga tcctcaactc ggtgctgcgc gacttctcac tgcagcagat caactccgtg     420

tgcgatgagc tgcacatcta ctgctcgcgc tgcaccgccg accagctgga gatcctcaaa     480

gtcatgggca tcctgccctt ctccgcgccc tcctgcgggc tcatcaccaa gacggacgcc     540

<210> 2
<211> 12
<212> DNA
<213> Artificial

<220>
<223> synthetic oligonucleotide

<400> 2
ggtgcgcata tg 12


<210> 3
<211> 79
<212> DNA
<213> Artificial

<220>
<223> synthetic oligonucleotide

<400> 3
ctgcagctcg agatgcatgt cgcggccgcc tccgcggccg cctgatgagt ccgtgaggac 60

gaaacatgca taggctagc 79


<210> 4
<211> 47
<212> DNA
<213> Artificial

<220>
<223> synthetic oligonucleotide

<400> 4
ggcccttatc agggcctctg gccttggggg agggggaggc cagaatg 47


<210> 5
<211> 12
<212> DNA
<213> Artificial

<220>
<223>  synthetic oligonucleotide

<400>  5
tttaccgggc cc                                                          12


<210>  6
<211>  12
<212>  DNA
<213>  Artificial

<220>
<223>  synthetic oligonucleotide

<400>  6
tatcggcata tg                                                          12


<210>  7
<211>  20
<212>  DNA
<213>  Artificial

<220>
<223>  synthetic oligonucleotide

<400>  7
ccgcctgatg agtccgtgag                                                  20


<210>  8
<211>  19
<212>  DNA
<213>  Artificial

<220>
<223>  synthetic oligonucleotide

<400>  8
atcgaagtca tggtggcta                                                   19

<210> 9
<211> 18
<212> DNA
<213> Artificial

<220>
<223> synthetic oligonucleotide

<400> 9
gacgaaacat gcataggc 18